Europäisches Patentamt

**European Patent Office**

**Office européen des brevets**

(11) Veröffentlichungsnummer: **0 120 221**
**B1**

(12) **EUROPÄISCHE PATENTSCHRIFT**

(45) Veröffentlichungstag der Patentschrift:
**15.07.87**

(51) Int. Cl.⁴: **C 07 D 209/08**

(21) Anmeldenummer: **84100966.5**

(22) Anmeldetag: **31.01.84**

(54) **Verfahren zur Herstellung von 7-Alkylindolen.**

(30) Priorität: **23.03.83 CH 1584/83**

(43) Veröffentlichungstag der Anmeldung:
**03.10.84 Patentblatt 84/40**

(45) Bekanntmachung des Hinweises auf die Patenterteilung:
**15.07.87 Patentblatt 87/29**

(84) Benannte Vertragsstaaten:
**AT BE CH DE FR GB IT LI NL SE**

(56) Entgegenhaltungen:
**DE - C - 2 224 556**
**FR - A - 2 257 579**
**US - A - 2 891 965**
**US - A - 3 441 569**
**US - A - 3 894 042**

(73) Patentinhaber: **LONZA AG, Gampel/Wallis (CH)**

(72) Erfinder: **Hardt, Peter, Dr., Bäretstrasse 8, Visp (Kanton Wallis) (CH)**

(74) Vertreter: **von Füner, Alexander, Dr. et al, Patentanwälte v. Füner, Ebbinghaus, Finck Mariahilfplatz 2 & 3, D-8000 München 90 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bekannt, aus 2-Ethylanilin mit Hilfe von Dehydrierungskatalysatoren bei höheren Temperaturen zwischen 500 und 700 °C Indol herzustellen.

Nach DE-OS 20 49 752 und DE-OS 21 48 961 wird 2-Ethylanilin in Gegenwart von Wasser und Sauerstoff oder eines Sauerstoff enthaltenden Gases zu Indol umgesetzt. Es konnte später gezeigt werden, dass eine entsprechende Dehydrocyclisierung in einem vereinfachten Verfahren ohne Verwendung von Sauerstoff oder eines Sauerstoff enthaltenden Gases mit besseren Raum-Zeit-Ausbeuten durchgeführt werden kann (CH-PS 589 054).

7-Alkylindole sind durch katalytische Dehydrocyclisierung der entsprechenden 2,6-Dialkylaniline zugänglich, doch im Gegensatz zu Indol selbst nur mit geringer Selektivität. So ist aus 2,6-Diethylanilin 7-Ethylindol nur mit 40% Selektivität erhalten worden (US-PS 3 441 569). Andere Darstellungsverfahren für 7-Alkylindole sind technisch aufwendig oder verlaufen wenig selektiv. So kann 7-Ethylindol aus 2-Ethylanilin und Chloracetonitril mit Hilfe von AlCl$_3$, allerdings nur mit 20% Ausbeute, erhalten werden (J. Med. Chem. 1980, 23, No. 11, S. 1224). Bei der katalytischen Dehydrocyclisierung von 2,6-Diethylanilin in Gegenwart von Wasser (CH-PS 589 054) wird eine Mischung von verschiedensten Verbindungen erhalten. Das darin als Hauptkomponente enthaltene 7-Ethylindol kann nicht ohne weiteres durch Destillation gewonnen werden, da die vinylgruppenhaltigen Nebenprodukte zu spontanen Polymerisationsreaktionen neigen und dadurch zu technischen Problemen, hohen Substanzverlusten und Qualitätseinbussen Anlass geben.

Aus der US-PS 3 894 042 ist bekannt, 7-Ethylindol durch katalytische Umsetzung von 2,6-Diethylanilin in Gegenwart von Wasserdampf unter Sauerstoffausschluss in einer per-pass Ausbeute von bescheidenen 13,2 Mol-% herzustellen.

Ziel der Erfindung ist es, die Nachteile der bekannten Verfahren zu vermeiden und insbesondere die Ausbeute zu erhöhen.

Erreicht wird dies durch ein Verfahren gemäss Patentanspruch 1, nämlich durch ein Verfahren zur Herstellung von 7-Ethylindol durch katalytische Dehydrocyclisierung von 2,6-Diethylanilin in Gegenwart von Wasserdampf ohne Zusatz von Sauerstoff oder einem Sauerstoff enthaltenden Gas in Gegenwart eines Katalysators, das dadurch gekennzeichnet ist, dass in Gegenwart eines Kupferchromitkatalysators, der mit Bariumoxid aktiviert ist, bei Temperaturen zwischen 500 und 700 °C und einem Druck zwischen 0,013 und 10 bar gearbeitet wird, wobei das Molverhältnis von Wasserdampf zum Ausgangsprodukt innerhalb des Bereiches von 3 zu 1 bis 75 zu 1 variieren kann und das erhaltene Rohprodukt in einer weiteren Stufe einer partiellen Hydrierung mittels eines Hydrierungskatalysators unterzogen wird.

Nach einer bevorzugten Ausführungsform erfolgt die katalytische partielle Hydrierung bei Temperaturen von 40 bis 120 °C. Bevorzugt wird die partielle Hydrierung unter einem H$_2$-Druck von 4 bis 12 bar durchgeführt. Es ist weiter bevorzugt, als Hydrierungskatalysator Raney-Nickel oder Edelmetalle einzusetzen.

Der Katalysator kann sowohl in einem Festbett als auch in einem sich bewegenden oder in einem Wirbelbett verwendet werden. Der Katalysator verliert seine Wirksamkeit praktisch nicht und die Aktivität bleibt vollständig erhalten. Deshalb erübrigt sich auch eine Reaktivierung.

Es kann zusätzlich ein inertes Verdünnungsmittel verwendet werden, das aus der Gruppe Stickstoff, Argon, Kohlendioxid, gesättigte Kohlenwasserstoffe, wie z.B. n-Pentan, Isopentan, n-Hexan, N-Heptan oder irgendeiner anderen Substanz, die bei den Reaktionsbedingungen nicht verändert wird, ausgewählt werden kann. Das Molverhältnis von Wasserdampf zu dem aromatischen Ausgangsprodukt kann innerhalb des Bereiches von 3 zu 1 bis 75 zu 1 variiert werden.

Das erfindungsgemässe Verfahren wird bei einer Temperatur innerhalb des Bereiches von 500 bis 700 °C, vorzugsweise bei 550 bis 650 °C, durchgeführt. Der Reaktionsdruck wird innerhalb eines breiten Bereiches zwischen 0,013 und 10 bar variiert, wobei das Verfahren bevorzugt bei Atmosphärendruck durchgeführt wird.

Die scheinbare Kontaktzeit zwischen den Reaktionsteilnehmern und den Katalysatoren liegt innerhalb des Bereiches von 0,1 bis 60 Sekunden, wobei der Bereich von 0,5 bis 30 Sekunden besonders bevorzugt ist. Unter der scheinbaren Kontaktzeit zwischen den Reaktionsteilnehmern und dem Katalysator ist das Verhältnis zwischen dem Volumen des Katalysatorbettes und dem Strom der Reaktionsteilnehmer als das Gas bei den Reaktionsbedingungen zu verstehen.

Es wurde nun gefunden, dass durch eine im Anschluss an die katalytische Dehydrocyclisierung durchgeführte partielle Hydrierung die im Reaktionsgemisch vorliegenden Vinylgruppen zu den entsprechenden Ethylgruppen hydriert werden.

Die partielle Hydrierung des nach der Dehydrocyclisierung des 2,6-Diethylanilins anfallenden Rohproduktes erfolgt in einem Rührautoklaven unter Verwendung von Raney-Nickel als Hydrierungskatalysator oder einem Nickel-, Palladium-, Platin- oder Rutheniumkatalysator, welcher in Mengen von 0,1 bis 1%, vorzugsweise von 0,3 bis 0,6%, eingesetzt wird.

Die Hydrierung wird vorzugsweise bei 50 bis 60 °C durchgeführt.

Der bei der Hydrierung angelegte Druck beträgt vorzugsweise 5 bis 6 bar.

Die Zusammensetzung des Reaktionsgemisches wird dadurch vereinfacht und der prozentuale Anteil an 7-Ethylindol erhöht. Das 7-Ethylindol lässt sich ohne technische Probleme in hoher Reinheit isolieren.

7-Ethylindol stellt ein wichtiges Zwischenprodukt für die Herstellung von pharmazeutischen Wirkstoffen dar (J. Med. Chem. 1976, Vol 19, No. 3, S. 391, und «Drugs of Future», Vol. II, Nr. 1 (1977) S. 21) und wird durch dieses einfache erfindungsgemässe Verfahren in guter Ausbeute und hoher Reinheit zugänglich. So kann durch dieses einfach durchzuführende Darstellungsverfahren bei 78% 2,6-Diethylanilin-Umsatz 7-Ethylindol mit einer Selektivität von 67% erhalten werden.

In den folgenden Beispielen sind die Prozentangaben Gewichts-Prozente. Der Umsatz bezieht sich auf eingesetztes Edukt, die Selektivität ist Ausbeute und ist auf umgesetztes Edukt bezogen.

Beispiel 1
7-Ethylindol

Der Reaktor für die Dehydrocyclisierung enthielt 0,5 l Cu-Chromit/BaO-Katalysator in Tablettenform (4 × 4 mm) und wurde auf 660 °C erwärmt und mit 68 g 2,6-Diethylanilin und 81 g Wasser pro Stunde beschickt. Nach dem Abkühlen erhielt man in der Vorlage ein Gemisch aus einer schweren organischen und einer leichteren Wasserphase, sowie ein Abgas folgender typischer Zusammensetzung (Vol. %): 80,0% Wasserstoff; 2,9% Methan; 2,0% Ethan; 0,4% Ethylen; 2,0% Kohlenmonoxid; 10,7% Kohlendioxid.

Pro Stunde wurden 38 l Abgas, 59 g organisches Rohprodukt und 75 g Wasser, welches wieder verwendet werden kann, erhalten. Das organische Rohprodukt hatte folgende Zusammensetzung:

| | |
|---|---|
| 2-Ethylanilin | 2,4% |
| 2-Methyl-6-ethylanilin | 2,2% |
| 2,6-Diethylanilin | 22,4% |
| 2-Ethyl-6-vinylanilin | 6,4% |
| Indol | 1,8% |
| 7-Methylindol | 3,9% |
| 7-Ethylindol | 46,5% |
| 7-Vinylindol | 11,5% |

Diesen Ergebnissen entspricht ein Umsatz von 80,6% und eine Selektivität von 51,4%.

295 g des Rohproduktes wurden in einem Rührautoklaven nach Zusatz von 1,2 g Raney-Nickel bei 55 °C und einem Druck von 5 bar $H_2$ bis zur Sättigung hydriert.

Nach dem Abkühlen des Autoklaven auf 20 °C, Entspannen und Abfiltrieren des Katalysators wurden 290,5 g einer Flüssigkeit mit folgender Zusammensetzung erhalten:

| | |
|---|---|
| 2-Ethyl-6-ethylanilin | 2,5% |
| 2,6-Diethylanilin | 29,1% |
| Indol | 1,8% |
| 7-Methylindol | 3,8% |
| 7-Ethylindol | 58,2% |

Diesen Ergebnissen entspricht über die beiden Stufen, Dehydrocyclisierung und partielle Hydrierung, ein Umsatz von 75,1% und eine Selektivität von 68,0%.

Beispiel 2
7-Ethylindol

Der mit dem Cu-Chromit/BaO-Katalysator gefüllte Reaktor wurde auf 610 °C erwärmt und pro Stunde mit 68,4 g 2,6-Diethylanilin und 41,4 g Wasser beschickt. Das organische Reaktionsprodukt von 5 Versuchsstunden wurde gesammelt und einer partiellen Hydrierung, wie in Beispiel 1 beschrieben, unterworfen. Nach dem Abfiltrieren des Hydrierkatalysators wurden 332,4 g organische Flüssigkeit mit 45,8% 2,6-Diethylanilin und 41,0% 7-Ethylindol erhalten.

Beispiel 3
7-Ethylindol

Der Reaktor mit 0,5 l Cu-Chromit/BaO-Katalysator, der bereits insgesamt 200 Stunden verwendet worden war, wurde bei 650 °C mit 71,7 g 2,6-Diethylanilin und 79 g Wasser pro Stunde beschickt. Über einen Zeitraum von 8 Stunden wurden 521,8 g organische Phase erhalten. Diese wurde anschliessend, nach Zusatz von 0,8 g Raney-Nickel, das 10% Co enthielt, in einem Rührautoklaven bei 55 °C und 10 bar Wasserstoff bis zum Erreichen einer Sättigung hydriert. Nach dem Abkühlen, Entspannen und Abfiltrieren des Katalysators erhielt man 511,3 g einer organischen Flüssigkeit folgender Zusammensetzung:

| | |
|---|---|
| 2-Ethylanilin | 3,0% |
| 2-Methyl-6-ethylanilin | 2,4% |
| Indol | 2,0% |
| 2,6-Diethylanilin | 29,3% |
| 7-Methylindol | 3,5% |
| 7-Ethylindol | 57,9% |

Durch fraktionierende Vakuumdestillation wurden nach einem Vorlauf, einer Fraktion mit 2,6-Diethylanilin und einem Zwischenlauf bei 140 °C/10 mbar 267,5 g 98,3%iges 7-Ethylindol erhalten. Dieses Ergebnis entsprach 77,7% Umsatz und 66,8% Selektivität.

Beispiel 4
7-Ethylindol

442,5 g organisches Rohprodukt, das gemäss Beispiel 1 hergestellt worden war und

| | |
|---|---|
| 2,6-Diethylanilin | 22,6% |
| 2-Ethyl-6-vinylanilin | 7,2% |
| 7-Ethylindol | 44,2% |
| 7-Vinylindol | 12,9% |

enthielt, wurden in einem Rührautoklaven nach Zusatz von 2,5 g Platinkatalysator (5% auf A-Kohle) bei 55 °C und 6 bar mit Wasserstoff gesättigt. Dazu waren ca. 4 Stunden erforderlich. Anschliessend wurde der Autoklav abgekühlt und entspannt. Nach dem Abtrennen des Katalysators durch Dekantieren und Zentrifugieren wurden 440,4 g Reaktionsprodukt erhalten, das zu 29,5% aus 2,6-Diethylanilin und zu 57,1% aus 7-Ethylindol bestand.

Beispiel 5
7-Ethylindol

Analog Beispiel 4 wurden 442,5 g organisches Rohprodukt mit 1,5 g Palladiumkatalysator (5% auf A-Kohle) hydriert. Nach 6 Stunden war die Reaktion beendet. Es wurden 438,0 g Reaktionsprodukt erhalten, in welchem keine vinylgruppenhaltigen Verbindungen mehr nachweisbar waren.

## Beispiel 6
### 7-Ethylindol

Analog Beispiel 4 wurden 442,5 g Rohprodukt mit 2,5 g Rutheniumkatalysator (5% auf A-Kohle) bei 55 °C und 10 bar Wasserstoffdruck hydriert. Im Reaktionsprodukt waren keine vinylgruppenhaltigen Verbindungen mehr nachzuweisen.

## Beispiel 7
### 7-Ethylindol

Analog Beispiel 4 wurden 426 g Rohprodukt mit 1,1 g Nickelkatalysator während 8 Stunden bei 55 °C und 10 bar hydriert. Im Reaktionsprodukt war kein 2-Ethyl -6- vinylanilin enthalten und der Gehalt an 7-Vinylindol war von 12,9% auf 0,4% zurückgegangen.

## Patentansprüche

1. Verfahren zur Herstellung von 7-Ethylindol durch katalytische Dehydrocyclisierung von 2,6-Diethylanilin in Gegenwart von Wasserdampf ohne Zusatz von Sauerstoff oder einem Sauerstoff enthaltenden Gas in Gegenwart eines Katalysators, dadurch gekennzeichnet, dass in Gegenwart eines Kupferchromitkatalysators, der mit Bariumoxid aktiviert ist, bei Temperaturen zwischen 500 und 700 °C und einem Druck zwischen 0,013 und 10 bar gearbeitet wird, wobei das Molverhältnis von Wasserdampf zum Ausgangsprodukt innerhalb des Bereiches von 3 zu 1 bis 75 zu 1 variieren kann und das erhaltene Rohprodukt in einer weiteren Stufe einer partiellen Hydrierung mittels eines Hydrierungskatalysators unterzogen wird.

2. Verfahren nach Patentanspruch 1, dadurch gekennzeichnet, dass die katalytische partielle Hydrierung bei Temperaturen von 40 bis 120 °C durchgeführt wird.

3. Verfahren nach Patentansprüchen 1 und 2, dadurch gekennzeichnet, dass die partielle Hydrierung unter einem $H_2$-Druck von 4 bis 12 bar durchgeführt wird.

4. Verfahren nach Patentanspruch 1 bis 3, dadurch gekennzeichnet, dass als Hydrierungskatalysator Raney-Nickel eingesetzt wird.

5. Verfahren nach Patentansprüchen 1 bis 3, dadurch gekennzeichnet, dass als Hydrierungskatalysatoren Edelmetalle verwendet werden.

## Revendications

1. Procédé pour la préparation du 7-éthylindole par déshydrocyclisation catalytique de la 2,6-diéthylaniline en présence de vapeur d'eau sans addition d'oxygène ou d'un gaz contenant de l'oxygène en présence d'un catalyseur, caractérisé en ce que l'on travaille en présence d'un catalyseur en chromite de cuivre qui est activé par de l'oxyde de baryum, à des températures entre 500 et 700 °C et sous une pression entre 0,013 et 10 bars, le rapport molaire de la vapeur d'eau au produit de départ pouvant varier à l'intérieur du domaine de 3 à 1 jusqu'à 75 à 1 et en ce que le produit brut obtenu est soumis, dans une étape supplémentaire, à une hydrogénation partielle au moyen d'un catalyseur d'hydrogénation.

2. Procédé selon la revendication 1, caractérisé en ce que l'hydrogénation catalytique partielle est effectuée à des températures de 40 à 120 °C.

3. Procédé selon les revendications 1 et 2, caractérisé en ce que l'hydrogénation partielle est effectuée sous une pression de $H_2$ de 4 à 12 bars.

4. Procédé selon les revendications 1 à 3, caractérisé en ce qu'on utilise du nickel de Raney comme catalyseur d'hydrogénation.

5. Procédé selon les revendications 1 à 3, caractérisé en ce qu'on utilise des métaux nobles comme catalyseurs d'hydrogénation.

## Claims

1. Process for the preparation of 7-ethylindole by catalytic dehydrocyclisation of 2,6-diethylaniline in the presence of water vapour without the addition of oxygen or of an oxygen-containing gas in the presence of a catalyst, characterised in that one works in the presence of a copper chromite catalyst which is activated with barium oxide at temperatures between 500 and 700 °C and a pressure between 0,013 and 10 bar, whereby the mole ratio of water vapour to the starting product can vary within the range of 3 to 1 to 75 to 1 and the crude product obtained is subjected, in a further step, to a partial hydrogenation by means of a hydrogenation catalyst.

2. Process according to patent claim 1, characterised in that the catalytic partial hydrogenation is carried out at temperatures of 40 to 120 °C.

3. Process according to patent claims 1 and 2, charaterised in that the partial hydrogenation is carried out at an $H_2$ pressure of 4 to 12 bar.

4. Process according to patent claims 1 to 3, characterised in that Raney nickel is used as hydrogenation catalyst.

5. Process according to patent claims 1 to 3, characterised in that noble metals are used as hydrogenation catalysts.